# EUROPEAN PATENT SPECIFICATION

(11) **EP 0 987 235 B1**
(45) Date of publication and mention of the grant of the patent: **12.03.2003**
(21) Application number: 99116091.2
(22) Date of filing: 17.08.1999
(51) Int. Cl.: C07B 37/10, C07B 37/04

(54) **Method for the conversion of arenes or alkenes with iodoalkenes, aryl iodides or arenediazonium salts**
Verfahren zur Umsetzung von Arenen oder Alkenen mit Iodalkenen, Aryliodiden oder Arendiazoniumsalzen
Procédé pour la conversion d'arènes ou d'alkènes avec des iodoalkènes, des aryliodides ou des sels arènediazonium

(30) Priority: 25.08.1998 EP 98115971
(43) Date of publication of application: 22.03.2000
(73) Proprietor: MERCK PATENT GmbH, 64293 Darmstadt (DE)
(72) Inventor: Murphy, John, Prof., Craigmarloch, Cumbernold G68 0GN (GB); Graham, Stephen, Glasgow G12 8RN (GB)

(56) References cited:
- WO-A-97/42194
- D. H. R. BARTON: "The invention of radical reactions. 32. Radical deoxygenations, dehalogenations, and deaminations with dialkyl phosphites and hypophosphorous acid as hydrogen sources" JOURNAL OF ORGANIC CHEMISTRY, vol. 58, no. 24, 19 November 1993 (1993-11-19), pages 6838-6842, XP002122647 EASTON US
- C. CHATGILIALOGLU: "Organosilanes as radical-based reducing agents in synthesis" ACCOUNTS OF CHEMICAL RESEARCH., vol. 25, no. 4, April 1992 (1992-04), pages 188-194, XP002122648 AMERICAN CHEMICAL SOCIETY. WASHINGTON., US ISSN: 0001-4842
- S. R. GRAHAM: "Hypophosphite mediated carbon-carbon bond formation: a clean approach to radical methodology" TETRAHEDRON LETTERS, vol. 40, no. 12, 19 March 1999 (1999-03-19), pages 2415-2416, XP002122649 OXFORD GB

## Description

The invention relates to a method for the conversion of alkenes or arenes with iodoalkenes, aryl iodides or arenediazonium salts in the presence of hypophosphorous acid or its derivatives and a radical initiator.

The inventive procedure is based on a typical radical reaction. These reactions are widely used in the formation of carbon-carbon bonds. There have been a number of useful procedures developed for reaction of radical intermediates. The key step in these procedures involves addition of a radical center to an unsaturated functional group. The radical formed by the reaction must then give rise to a new radical which can propagate the chain. An important group of such reactions involves halides as the source of the radical intermediate. The radicals are normally generated by halogen atom abstraction with a trialkylstannane as the reagent and an initiator such as azoisobutyronitrile (AIBN) or dibenzoylperoxide. The majority of radical reactions are based on tin hydrides as reducing agents and chain carriers, mainly tri-n-butyltin hydride (M. Pereyre, J.P.Quintard, A. Rahm, Tin in Organic Synthesis, Butterworths, London, 1986). However, organotin compounds are toxic and expensive and are difficult to remove completely from the desired reaction products. Alternative reagents such as tris(trimethylsilyl)silane (C. Chatgiliaoglu, Acc. Chem. Res., 1992, 25, 188) have been proposed, but these compounds are generally too expensive for carrying out radical reactions on an industrial scale.

It is known that radical reactions that replace certain functional groups by hydrogen, such as decarboxylations, deoxygenations, deaminations and dehalogenations are easily performed in the presence of tri-n-butyltin hydride. The reaction can also be carried out effectively when using commercially available and inexpensive hypophosphorous acid, its salts or dialkylphosphites, thus circumventing the disadvantages of tri-n-butyltin hydride (D. H. R. Barton, D. O. Jang, J. C. Jaszberenyi, J. Org. Chem. 1993, 58, 6838-6842, Tetrahedron Letters, 33, 39, 5709-5712, D. O. Jang. Tetrahedron Letters, 37, 30, 5367-5368, 1996).

The document WO 97/42194 discloses the process wherein an unsaturated iodine containing compound is cyclized in a carbon-carbon-bond forming reaction can be carried out using hypophosphorous acid, azoisobutyronitrile in 1,4-dioxane.

The document Acc. Chem. Res. 1992, 25, p. 188-194 discloses on p. 192, left hand column, reaction (6), the reaction of cyclohexyliodide with methylacrylate with the formation of a carbon-carbon bond.

However, there is still a demand to achieve carbon-carbon bond formation via radical reactions without the aid of expensive and toxic reagents such as tri-n-butyltin hydride or tris(trimethylsilyl)silane.

It was now found that hypophosphorous acid and its derivatives can also be used efficiently in place of of tri-n-butyltin hydride or tris(trimethylsilyl)-silane to achieve carbon-carbon bond formation via radical reactions.

Thus, the invention relates to a method for the conversion of alkenes or arenes with iodoalkenes, aryl iodides or arenediazonium salts in the presence of hypophosphorous acid or its derivatives and a radical initiator.

In particular, the invention relates to a method for the conversion of alkenes or arenes with iodoalkenes, aryl iodides or arenediazonium salts in the presence of hypophosphorous acid or its derivatives and a radical initiator, characterized in that the reacting alkenes and/or arenes are part of the same compound, thus performing an intramolecular carbon-carbon bond formation.

Alkenes also include heteroalkenes, preferably azaalkenes such as oximes. Arenes include heteroarenes such as pyridines or pyrimidines.

The expression diazonium, diazonium group or diazonium salt refers to all known diazonium salts such as e.g. diazonium tetrafluoroborate or chloride.

The radical reaction of the inventive procedure is assumed to start with hydrogen abstraction of the hypophosphorous acid or its derivative by the radical initiator as exemplified in the following scheme:

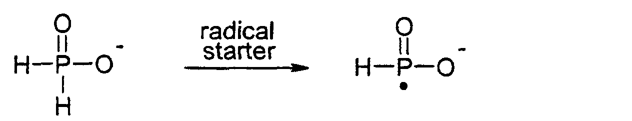

The radical then reacts with the iodoalkene or arene that is substituted by an iodo or a diazonium group. The organic radical thus formed adds to a double bond of an alkene or arene following known mechanisms:

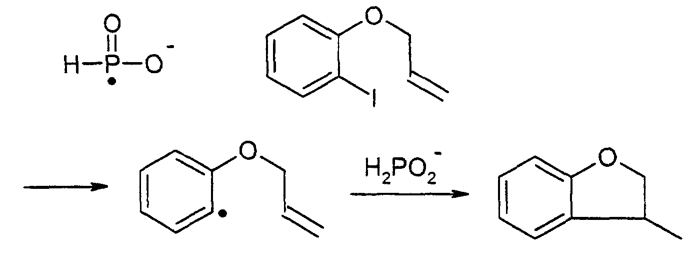

The invention especially relates to a process for the manufacture of compounds of formulae I and II 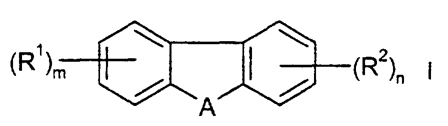 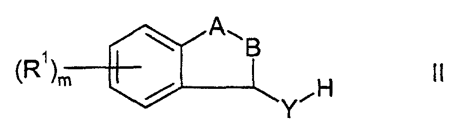 wherein
- R¹, R²: are each, independently of one another, H, F, Br, Cl, CN, NO₂, N(R³)₂, OR³, SR³, COOR³, NHCOR³, SF₅, SO₂R³ or Alkyl having 1 to 12 carbon atoms which is unsubstituted or at least monosubstituted by F, Br, Cl, CF₃ or CN, wherein it is also possible for one or more non-adjacent -CH₂-groups to be replaced, independently of one another, by -CH=CH-, -O-CH=CH-, -CH=CH-O-, -O-, -S-, -CO-, -O-CO- or -CO-O- or a mesogenic group,
- R³: H, alkyl or alkoxy having 1 to 12 carbon atoms or phenyl in which one or two CH-groups may also be replaced by N, which is unsubstituted or at least monosubstituted by F, Br, Cl, CF₃, CN, NO₂ or alkyl or alkoxy having 1 to 12 carbon atoms,
- A, B: are independently of one another, -O-, -S-, -N(R³)-, -N=C(R⁴)-, -C(R⁴)=N-, -C(R⁴)(R⁵)-, -C(R⁴)=C(R⁵)-, -C(R⁴)(R⁵)C(R⁶)(R⁷)-, -C(R⁴)(R⁵)-O-, -O-C(R⁴)(R⁵)-, -C(R⁴)(R⁵)-N(R³)- or -N(R³)-C(R⁴)(R⁵)-, in such a way that heteroatoms are not linked directly to one another,
- R⁴, R⁵, R⁶, R⁷: have independently of one another the meaning given for R¹ and R²,
- Y: CR⁴R⁵ or NR³
- m,n: 1, 2, 3 or 4,
by intramolecular reaction of the starting compounds III and IV 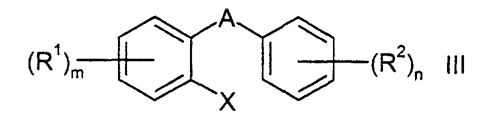 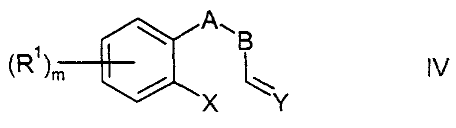 wherein
- X: is iodo or diazonium salt,
and
R¹, R², A, B, Y, m and n have the meaning given above, in the presence of hypophosphorous acid or its derivatives and a radical initiator.

In a preferred embodiment of the invention R¹ and/or R² have the meaning of a mesogenic group of formula V

R⁰-A¹-Z¹-(A²-Z²-)ₚ- V

wherein
- R⁰: H, F, Br, Cl, CN, NO₂, SF₅ or Alkyl having 1 to 12 carbon atoms which is unsubstituted or at least monosubstituted by F, Br, Cl, CF₃ or CN, wherein it is also possible for one or more non- adjacent -CH₂-groups to be replaced, independently of one another, by -CH=CH-, -O-CH=CH-, -CH=CH-O-, -O-, -S-, -CO-, -O-CO- or -CO-O-,
- A¹, A²: are each, independently of one another,
a) trans-1,4-cyclohexylene in which one or two non-adjacent CH-groups may also be replaced by O or S,
b) 1,4-phenylene in which one or two CH-groups may also be replaced by N,
c) 1,3-cyclobutylene, 1,3-bicyclo(1,1,1)pentylene, 1,4-cyclohexenylene, 1,4-bicyclo(2,2,2)octylene, piperidine-1,4-diyl, naphthalene-2,6-diyl, decahydronaphthalene-2,6-diyl or 1,2,3,4-tetrahydronaphthalene-2,6-diyl,
wherein groups under a) and b) may be substituted by CN, F, Cl or Br,
- Z¹, Z²: are each, independently of one another, -CH₂CH₂-, -C≡C-, -CH₂O-, -OCH₂-, -CO-O-, -O-CO-, -CH=N-, -N=CH-, -CH₂S- or -SCH₂-,
and
- p: is 0, 1, 2 or 3.

Above and below, R⁰, R¹, R², R³, R⁴, R⁵, R⁶, R⁷, A, B, Y, X, A¹, A², Z¹,Z², p, m and n are as defined above, unless expressly stated otherwise. If the group R¹ appears more than once, it can have identical or different meanings. The same applies to all other groups appearing more than once.

In compounds of formula I R¹ and/or R² are preferably Br, CN, NO₂, N(R³)₂, OR³, SR³, COOR³, NHCOR³, SO₂R³ or Alkyl having 1 to 12 carbon atoms which is unsubstituted wherein it is also possible for one or more non-adjacent -CH₂-groups to be replaced, independently of one another, by -O-, -S-, -CO-, -O-CO- or -CO-O- or a mesogenic group of formula V. In especially preferred compounds of formula I R¹ and/or R² are Br, CN, NO₂, N(R³)₂, OR³, SR³, COOR³, NHCOR³, SO₂R³ or a mesogenic group of formula V.

In compounds of formula II R¹, R⁴, R⁵, R⁶ and R⁷ are preferably H, Br, CN, N(R³)₂, OR³, SR³, COOR³, or Alkyl having 1 to 7 carbon atoms.

The preferred meaning of R³ in compounds of formulae I and II is H or Alkyl or Alkoxy having 1 to 5 carbon atoms.

A is in compounds of formulae I and II preferably -O-, -S-, -N(R³)-, -C(R⁴)(R⁵)-, -C(R⁴)=C(R⁵)-, -C(R⁴)(R⁵)C(R⁶)(R⁷)-, -C(R⁴)(R⁵)-O- or -O-C(R⁴)(R⁵)-.
X is preferably iodo. Preferred meanings of m and n are 0, 1 or 2.

R⁰ is preferably F, CN, CF₃, OCF₃, CHF₂, OCF₂, CHF₂, OCHF₂, OCF₂CF₃, OCH₂CF₃, OCHF₂CF₃, SF₅ or Alkyl or Alkoxy, having 1 to 7 carbon atoms or Alkenyl or Alkenyloxy having 2 to 7 carbon atoms.

Preference is given to the compounds of the formulae I1 to I16 in the following group which can be obtained by the inventive process: 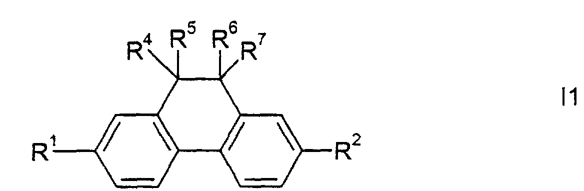 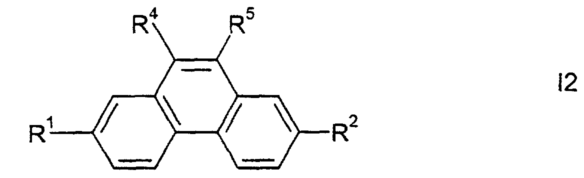 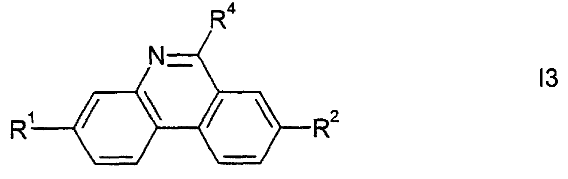 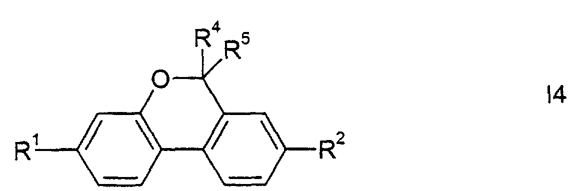 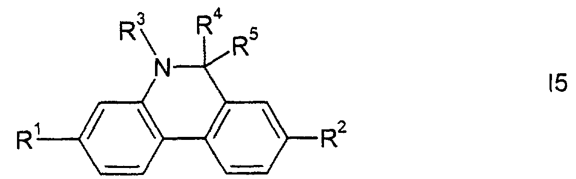 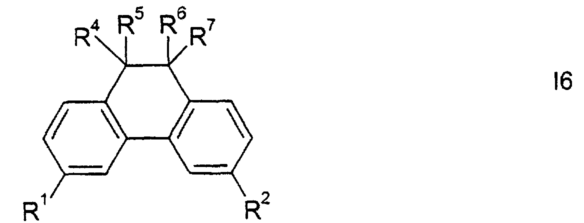 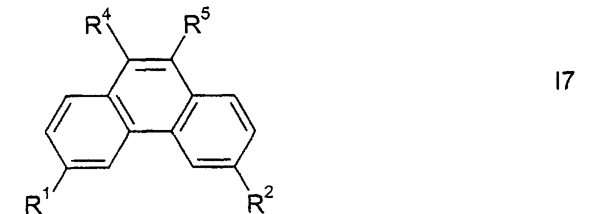 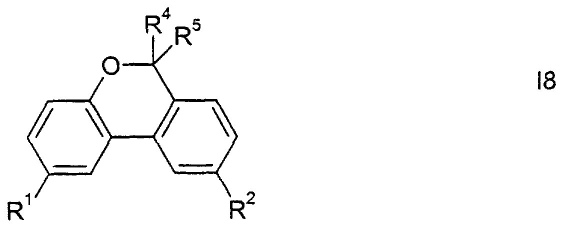 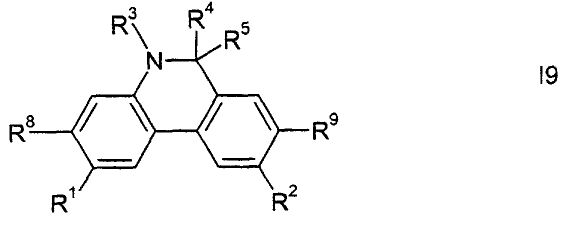 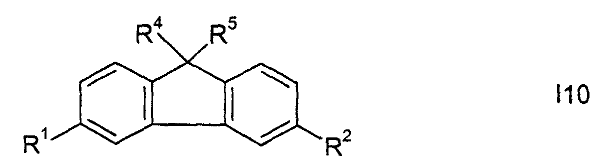 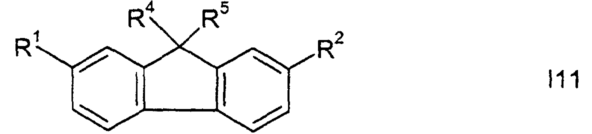 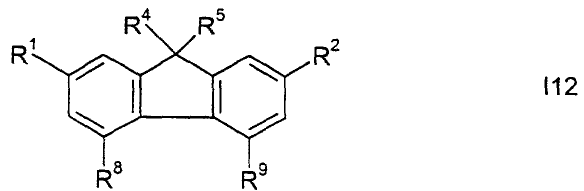 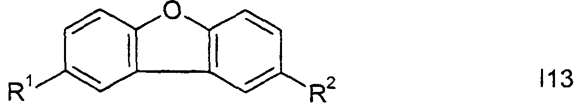 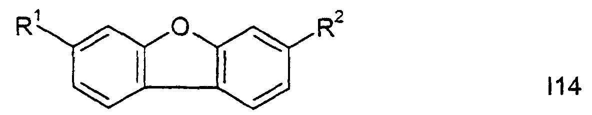 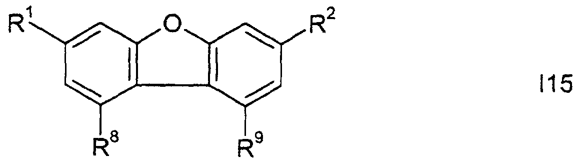 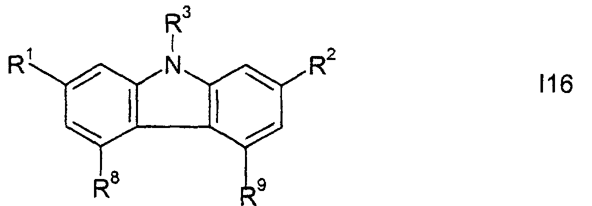 in which R¹, R², R³, R⁴, R⁵ and R⁶ are as defined above and R⁸ and R⁹ have the meaning given for R¹ and R².

Furthermore, preference is given to the compounds of the formulae II1 to II7 in the following group: 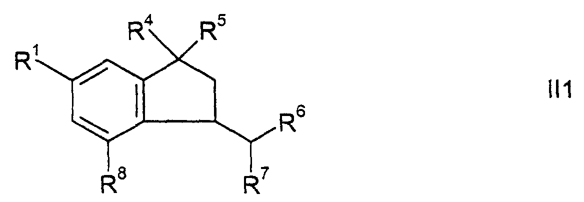 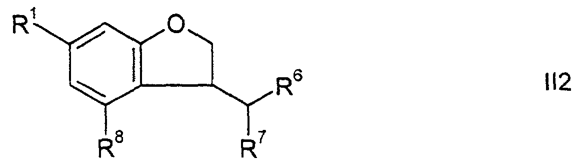 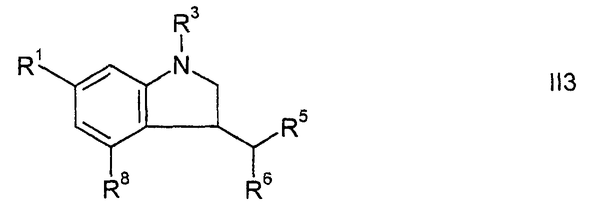 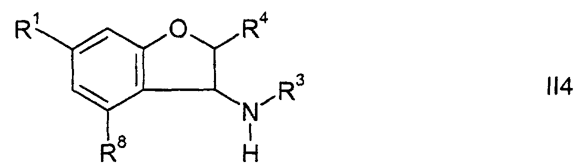 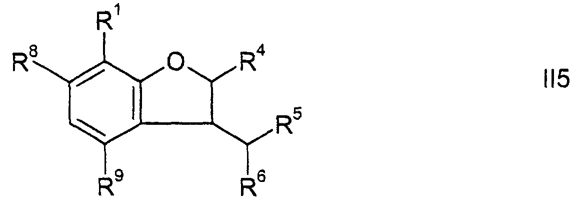 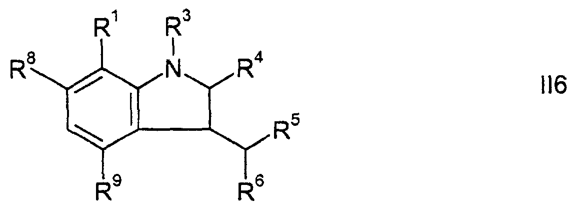 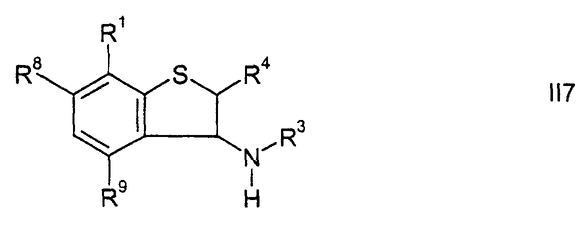 in which R¹, R², R³, R⁴, R⁵ and R⁶ are as defined above and R⁸ and R⁹ have the meaning given for R¹ and R².

Very particularly preferred compounds from these groups are those of formulae I1, I2, I3, I7, I10, II1, II2 and II6.

The compounds of formula I obtained in this way can be used for the synthesis of liquid crystal compounds and can readily be processed further to give a very wide variety of end products, e.g. by hydrogenation to give the corresponding saturated rings.

Compounds prepared by the inventive process may be used as products or intermediates in industrial organic chemistry, especially for applications in pharmaceutical or pesticide synthesis.

If R¹, R², R⁴, R⁵, R⁶, R⁷ or R⁰ in the formulae above and below is an alkyl group and/or alkoxy group, this can be straight-chain or branched. It is preferably straight-chain, has 2, 3, 4, 5, 6 or 7 carbon atoms and accordingly is preferably ethyl, propyl, butyl, pentyl, hexyl, heptyl, ethoxy, propoxy, butoxy, pentoxy, hexoxy or heptoxy, furthermore methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxymethyl), 2- (= ethoxymethyl) or 3-oxabutyl (= 2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, or 2-, 3-, 4-, 5-, 6-, 7-, 8- or 9-oxadecyl.

If R¹, R², R⁴, R⁵, R⁶, R⁷ or R⁰ is an alkyl group in which one CH₂ group has been replaced by -CH=CH-, this can be straight-chain or branched. It is preferably straight-chain and has 2 to 10 carbon atoms. Accordingly, it is in particular vinyl, prop-1- or -2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5-, -6- or -7-enyl, non-1-, -2-, -3-, -4-, -5-, -6-, -7- or -8-enyl, or dec-1-, -2-, -3-, -4-, -5-, -6-, -7-, -8- or -9-enyl. Alkenes having an E-double bond are preferred.

If R¹, R², R⁴, R⁵, R⁶, R⁷ or R⁰ is an alkyl group in which one CH₂ group has been replaced by -O- and one has been replaced by -CO-, these are preferably adjacent. These thus contain one acyloxy group -CO-O- or one oxycarbonyl group -O-CO-. These are preferably straight-chain and have 2 to 6 carbon atoms. Accordingly, they are in particular acetoxy, propionyloxy, butyryloxy, pentanoyloxy, hexanoyloxy, acetoxymethyl, propionyloxymethyl, butyryloxymethyl, pentanoyloxymethyl, 2-acetoxyethyl, 2-propionyloxyethyl, 2-butyryloxyethyl, 3-acetoxypropyl, 3-propionyloxypropyl, 4-acetoxybutyl, methoxycarbonyl, ethoxycarbonyl, propoxycarbonyl, butoxycarbonyl, pentoxycarbonyl, methoxycarbonylmethyl, ethoxycarbonylmethyl, propoxycarbonylmethyl, butoxycarbonylmethyl, 2-(methoxy-carbonyl)ethyl, 2-(ethoxycarbonyl)ethyl, 2-(propoxy-carbonyl)ethyl, 3-(methoxy-carbonyl)propyl, 3-(ethoxy-carbonyl)propyl or 4-(methoxycarbonyl)butyl.

If R¹, R², R⁴, R⁵, R⁶, R⁷ or R⁰ is an alkyl group in which one CH₂ group has been replaced by unsubstituted or substituted -CH=CH- and an adjacent CH₂ group has been replaced by CO or CO-O or O-CO-, this can be straight-chain or branched. It is preferably straight-chain and has 4 to 13 carbon atoms. Accordingly, it is in particular acryloyloxymethyl, 2-acryloyloxyethyl, 3-acryloyloxypropyl, 4-acryloyloxybutyl, 5-acryloyloxypentyl, 6-acryloyloxyhexyl, 7-acryloyloxyheptyl, 8-acryloyloxyoctyl, 9-acryloyloxynonyl, 10-acryloyloxydecyl, methacryloyloxymethyl, 2-methacryloyloxyethyl, 3-methacryloyloxypropyl, 4-methacryloyloxybutyl, 5-methacryloyloxypentyl, 6-methacryloyloxyhexyl, 7-methacryloyloxyheptyl, 8-methacryloyloxyoctyl and 9-methacryloyloxynonyl.

If R¹, R², R⁴, R⁵, R⁶, R⁷ or R⁰ is an alkyl or alkenyl group which is monosubstituted by CN or CF₃, this group is preferably straight-chain, and the substitution by CN or CF₃ is in the ω-position.

If R¹, R², R⁴, R⁵, R⁶, R⁷ or R⁰ is an alkyl or alkenyl group which is at least monosubstituted by halogen, this group is preferably straight-chain, and halogen is preferably F or Cl. In the case of multiple substitution, halogen is preferably F. The resulting groups also include perfluorinated groups. In the case of monosubstitution, the fluorine or chlorine substituent can be in any desired position, but is preferably in the ω-position.

Compounds of the formula I containing branched wing groups R¹, R², R⁴, R⁵, R⁶, R⁷ or R⁰ may occasionally be of importance due to better solubility in the customary liquid-crystalline base materials, but in particular as chiral dopes if they are optically active. Smectic compounds of this type are suitable as components for ferroelectric materials.

Branched groups of this type generally contain not more than one chain branch. Preferred branched groups R¹, R², R⁴, R⁵, R⁶, R⁷ or R⁰ are isopropyl, 2-butyl (= 1-methylpropyl), isobutyl (= 2-methylpropyl), 2-methylbutyl, isopentyl (= 3-methylbutyl), 2-methylpentyl, 3-methylpentyl, 2-ethylhexyl, 2-propylpentyl, isopropoxy, 2-methylpropoxy, 2-methylbutoxy, 3-methylbutoxy, 2-methylpentoxy, 3-methylpentoxy, 2-ethylhexoxy, 1-methylhexoxy and 1-methylheptoxy.

The formulae I and II cover the racemates of these compounds and the optical antipodes, and mixtures thereof.

Of these compounds of the formula I, II and the sub-formulae, preference is given to those in which at least one of the groups present has one of the preferred meanings indicated.

The inventive procedure can be carried out preferably by dissolving the starting compounds in a suitable solvent under an inert gas atmosphere, adding hypophosphorous acid or its derivatives and a radical initiator and heating the mixture at a temperature of 30 to 200°C, preferably at 40 to 150°C, especially preferred at 45 to 130° until the reaction proceeds. In a preferred embodiment of the invention the reaction mixture is heated to the boiling point of the respective solvent.

Derivatives of hypophosphorous acid which can be used instead or in combination with hypophosphorous acid include salts of hypophosphorous acid such as alkali salts like sodium or potassium salts or addition salts of hypophosphorous acid and nitrogen bases like ammonia, trialkylamines such as triethylamine, tributylamine or triisooctylamine piperidine, n-alkylpiperidine such as n-ethylpiperidine, pyridine, pyrrolidine, imidazole, piperazine, cyclohexylamine, 2-aminoethanol, triethanolamine, aniline, n,n-dialkylaniline such as n,n-dimethylaniline, DABCO (1,4-diazabicyclo[2.2.2]octane), DBN (1,5-diazabicyclo[4.3.0]non-5-ene) or DBU (1,8-Diazabicyclo[5.4.0]undec-7-ene). Also, dialkylphosphites such as dimethylphosphite, diethylphosphite, di-n-propylphosphite, di-isopropylphosphite, di-n-butylphosphite, di-sec-butylphosphite, dipentylphosphite, [1,3,2]Dioxaphosphinane or [1,3,2]Dioxaphospholane or dialkylphosphines such as dimethylphosphine, diethylphosphine, di-n-propylphosphine, di-isopropylphosphine, di-n-butylphosphine, di-sec-butylphosphine dipentylphosphine, phospholane or phosphinane may be used.

Preferred solvents used for the inventive process are water, alcohols such as methanol, ethanol, n-propanol, i-propanol, n-butanol, i-butanol or t-butanol, sulfoxides such as dimethylsulfoxide or sulfolane, amides such as n,n-dimethylformamide or n-methylpyrrolidone, nitriles such as acetonitrile, ketones such as acetone, butanone, methylisopropylketone or methylisobutylketone, ethers such as diethylether methyl-tert-butylether, dioxane or tetrahydrofuran or anisole, esters such as methylacetate, ethylacetate, propylacetate or butylacetate, aromatic hydrocarbons such as benzene, toluene, xylenes or mesitylene, saturated hydrocarbons such as pentane, hexane, heptane, octane or cyclohexane or halogenated hydrocarbons such as dichloromethane, trichloromethane, dichloroethylene, trichloroethylene or fluorinated analogues or mixtures of the above mentioned solvents. Especially preferred solvents are water, alcohols such as methanol, ethanol, n-propanol, i-propanol, n-butanol, i-butanol or t-butanol, sulfoxides such as dimethylsulfoxide or sulfolane, amides such as n,n-dimethylformamide or n-methylpyrrolidone and nitriles such as acetonitrile or their mixtures.
In a particulary preferred embodiment of the invention water or its mixtures with organic solvents is used.

The amount of solvent is not critical, in general 10 to 10000 g of solvent may be used for each g of starting material.

The inventive procedure is especially advantageous to be performed in water when using water souble starting materials or materials that can be made water soluble by protonation or deprotonation as in the case with amines or carboxylic acids or sulfonic amides.
When water or a mixture containing water is used as a solvent in the inventive procedure, it may be advantageous to add tensides to the reaction mixture in order to achieve a better solubility of the starting material and the reagents and a higher reaction rate.

Depending on the type of starting material it may be necessary to add acids such as formic, acetic, hydrochloric or sulfuric acid or bases such as carbonates like sodium or potassium carbonate or sodium or potassium hydrogencarbonate, hydroxides like sodium or potassium hydroxide or nitrogen containing bases such as ammonia, trialkylamines like triethylamine, tributylamine or triisooctylamine piperidine, n-alkylpiperidine like n-ethylpiperidine, pyridine, pyrrolidine, imidazole, piperazine, cyclohexylamine, 2-aminoethanol, triethanolamine, aniline, n,n-dialkylaniline like n,n-dimethylaniline, DABCO, DBN or DBU to the mixture of these compounds and the solvent in order to achieve a homogenous mixture and to ensure in the case when hypophosphorous acid or its salts are used, that the hypophosphite anion stays unprotonated to an extent sufficient to perform the reaction.

The ratio of the molar amount of hypophosphorous acid or its derivatives to the molar amount of starting material is generally between 1 to 1 and 20 to 1, preferably between 2 to 1 and 15 to 1. In an especially preferred embodiment the ratio is between 5 to 1 and 10 to 1.

Preferably between 0.01 and 1.0 equivalents of a radical initiator are used in relation to hypophosphorous acid.

The reaction is preferably carried out in an intramolecular manner, i.e. the reaction centres (the iodoalkene, aryl iodide or arenediazonium salt and a double-bond of an alkene or arene that is not substituted by iodo or diazonium group) are part of the same molecule. In the case when the inventive procedure is not performed in an intramolecular manner, it may be advantageous to use the alkene or arene which is not substituted by iodo or diazonium group as solvent, i.e. in a 10 to 1000-fold excess compared to the iodoalkene or iodo or diazonium substituted arene.

All known radical initiators can be employed in the inventive procedure. Preferred radical initiators are for example AIBN, dibenzoylperoxide, benzoylperoxide or di-tert-butylperoxide.

The time of reaction usually is in the range of one minute to 2 days, preferably between 0.1 and 20 hours.

The process according to the invention starts from readily accessible starting compounds which are prepared by methods known per se, as described in the literature (for example in the standard works such as Houben-Weyl, Methoden der Organischen Chemie [Methods of Organic Chemistry], Georg-Thieme-Verlag, Stuttgart), under reaction conditions which are known and suitable for said reactions.

Use can also be made here of variants which are known per se, but are not described here in greater detail.

In conclusion, hypophosphorous acid and its derivatives are effective radical reducing agents and are ideal alternatives to organiotin or silicon hydrides in carbon-carbon bond forming reactions. They are inexpensive and less toxic than organotin hydrides. A simple work-up procedure can be applied for the purification of the products. The excess reagents and hypophosphorous-containing byproducts are washed out from the reaction mixture after radical reaction.

The examples below are intended to illustrate the invention without representing a limitation.

### Example 1

A solution of O-allyl-3,5-diiodosalicylic acid (100 mg, 0,23 mmol, made by allylation of 3,5-diiodsalicylic acid methyl ester with allylic alcohol in the presence of triphenylphosphine and diisopropyl azodicarboxylate and subsequent saponification) in distilled water (5 ml) was treated with sodium hydrogen carbonate (193 mg, 2.3 mmol). This mixture was stirred in an inert atmosphere until a clear solution was obtained. Hypophosphorous acid (0.184 ml, 1.84 mmol) was added to this mixture. Once foaming had ceased the reaction was heated to reflux for 1 h. AIBN
(40 mg/0,138 mmol) was added as a radical initiator in two portions over 30 mins. After addition of the second portion of initiator the reaction was heated at reflux for a further 15 h. On cooling to room temperature the reaction mixture was basified to pH 10 using 2M sodium hydroxide solution and extracted into dichloromethane. The aqueous layer was then acidified to pH 1 and extracted with dichloromethane. These organic layers were combined and dried over anhydrous magnesium sulfate before filtering and evaporating to dryness in vacuo. This yielded the cyclised product 3-methyl-2,3-dihydro-benzofuran-7-carboxylic acid as a fine white solid.

Accordingly, the following compounds are prepared from the respective starting materials by the inventive procedure:

### Examples 2 to 17

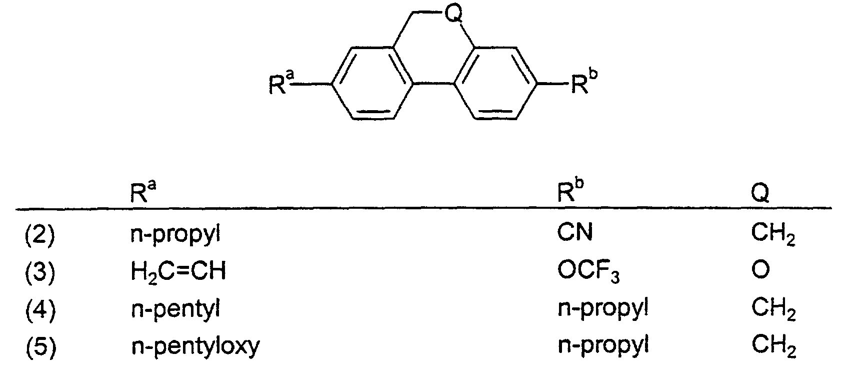

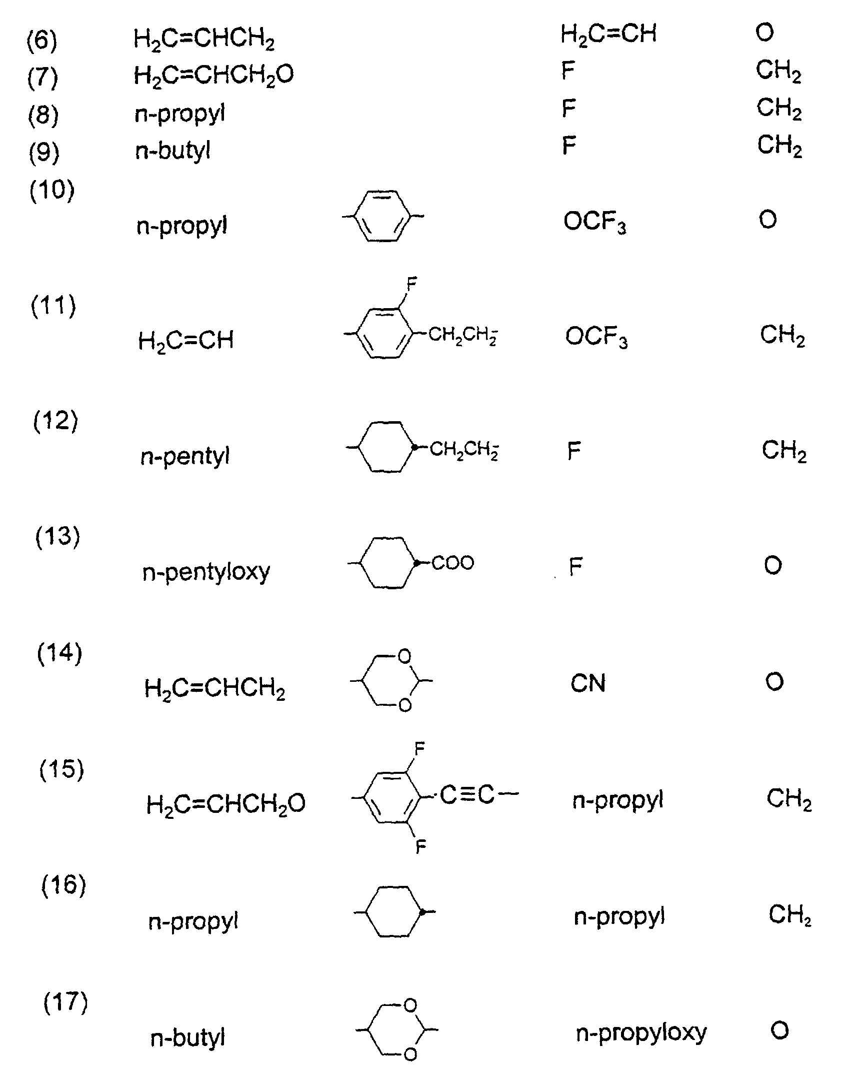

### Examples 18 to 33

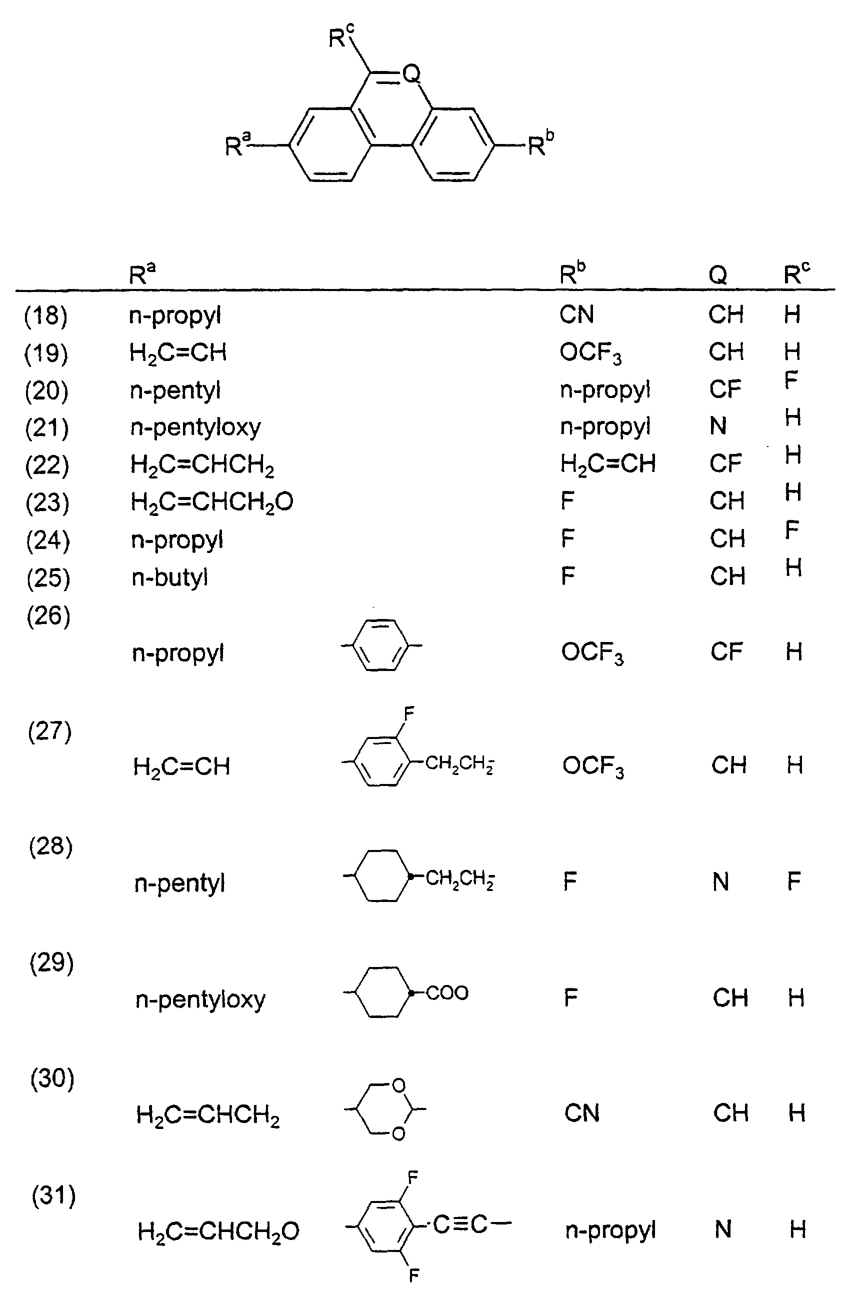

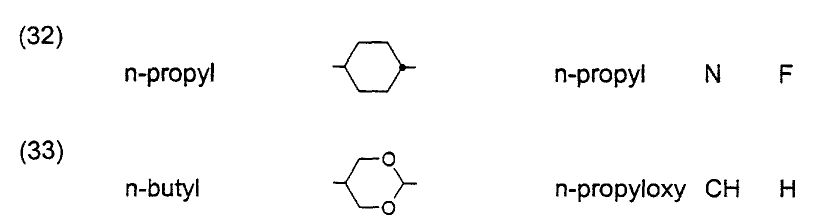

### Examples 34 to 49

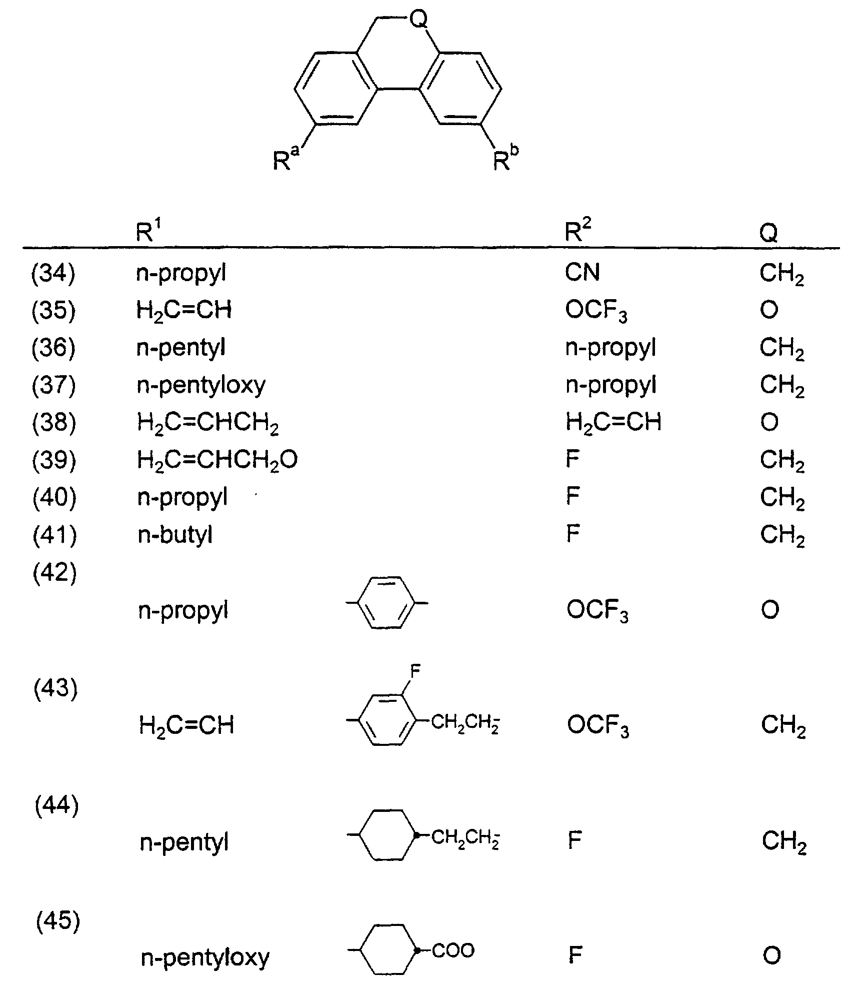

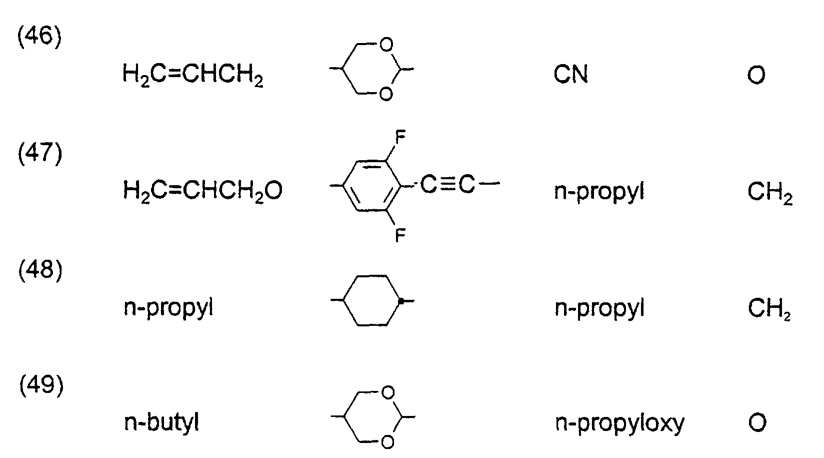

### Examples 50 to 65

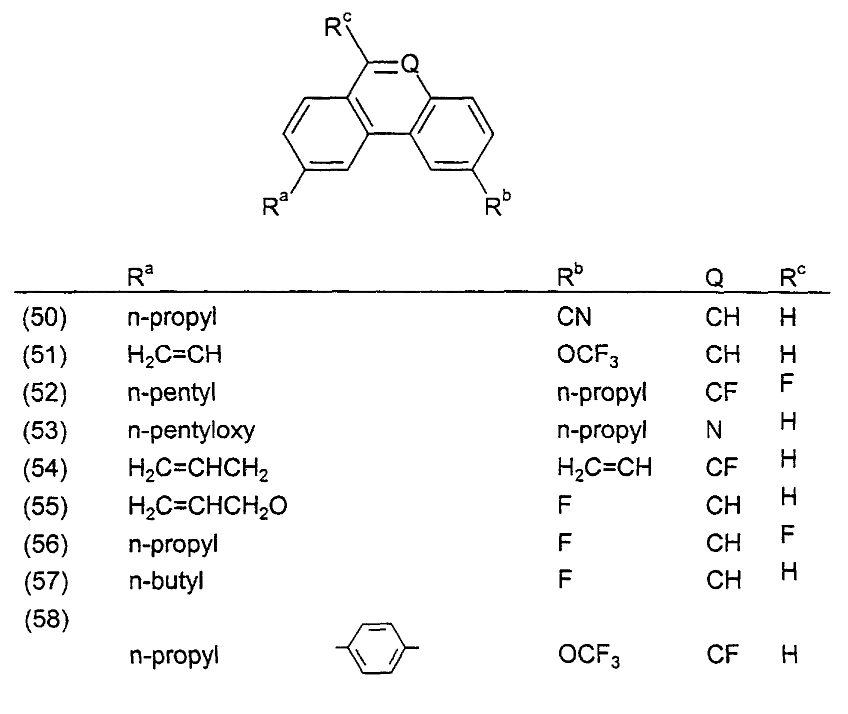

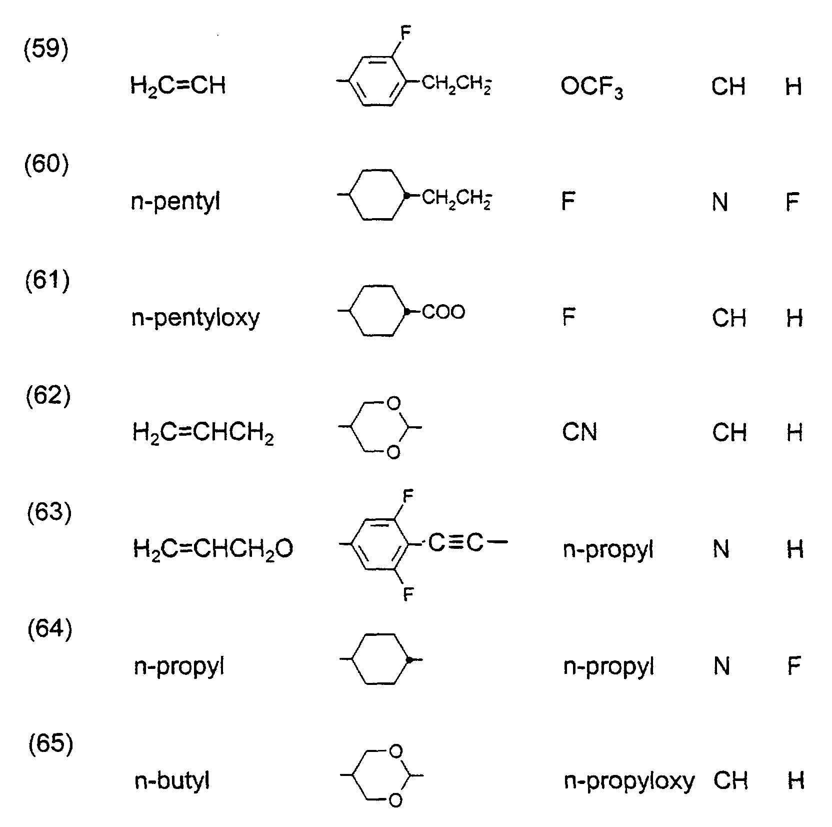

### Examples 66 to 81

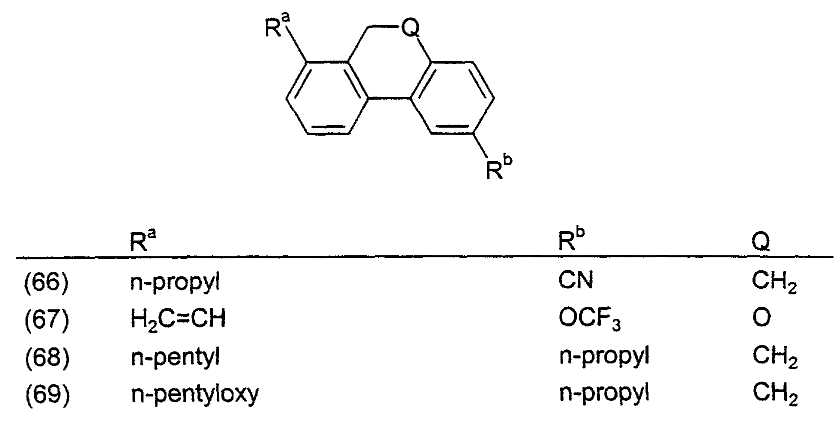

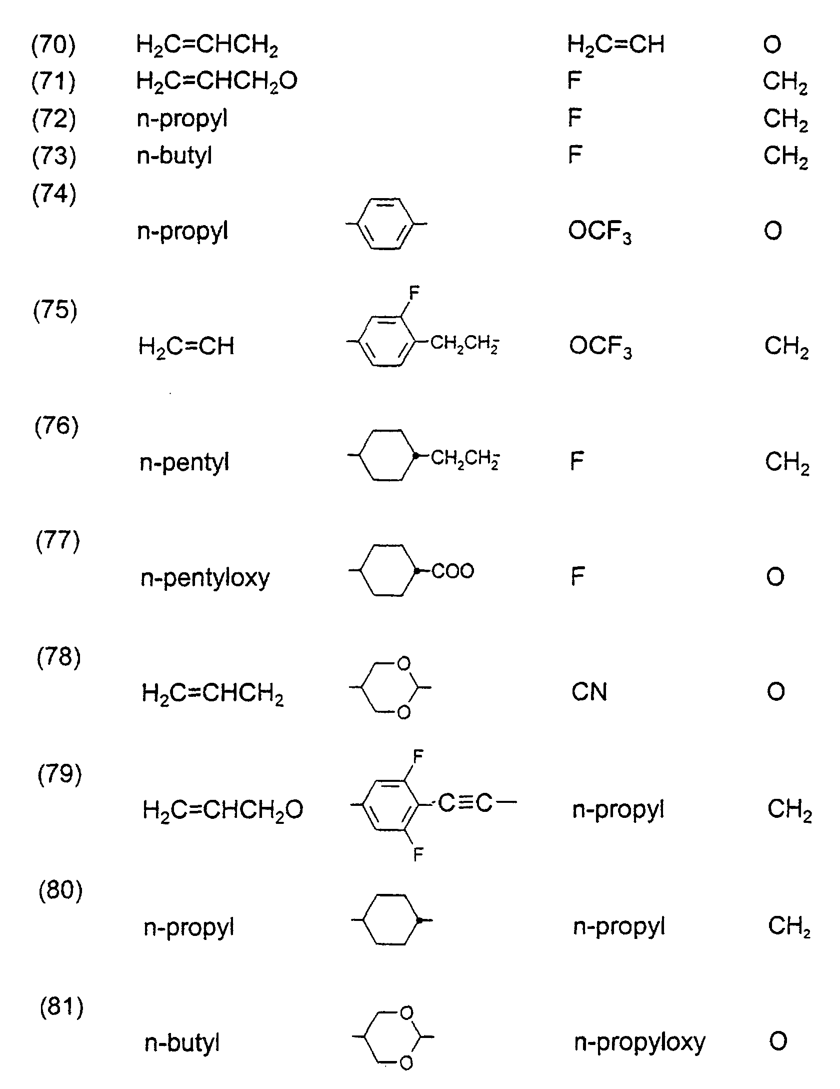

### Examples 82 to 97

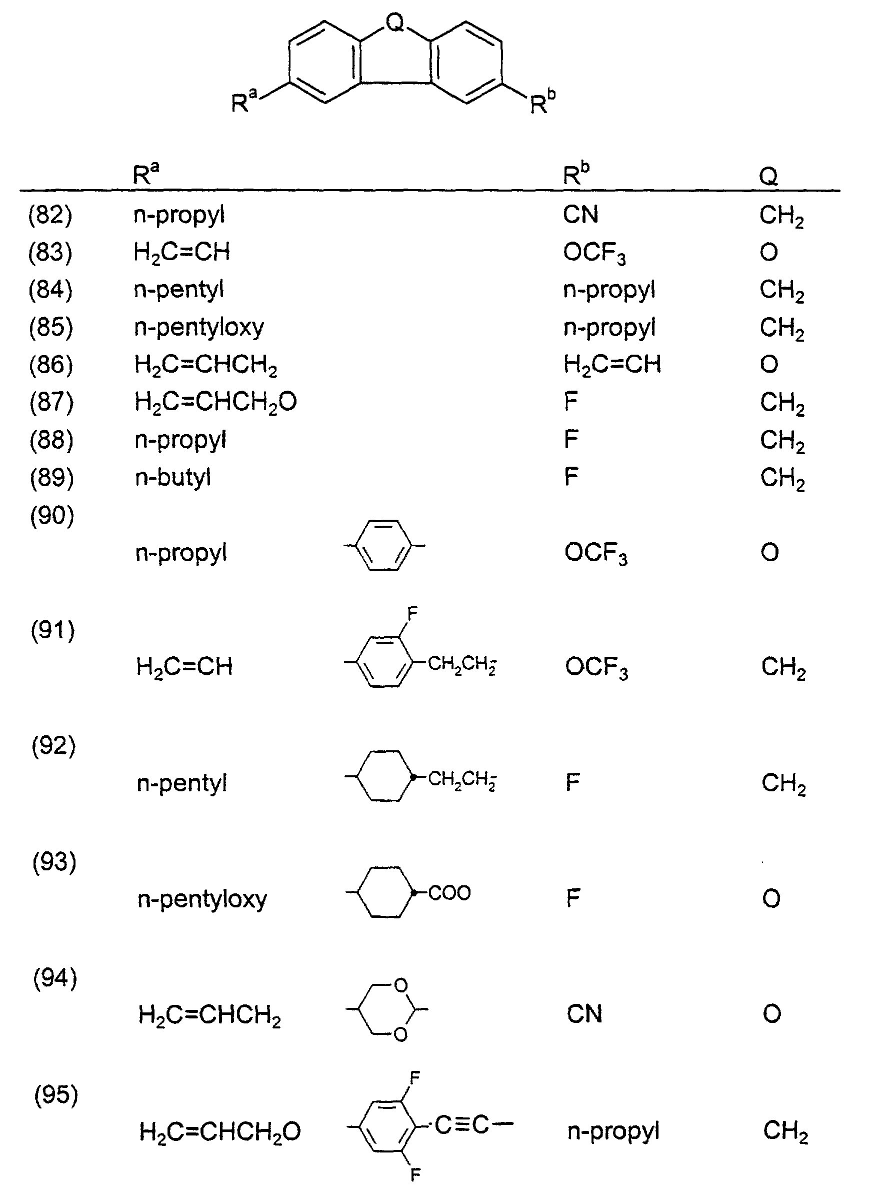

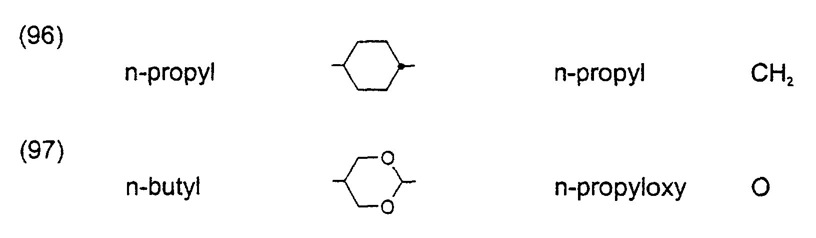

### Examples 98 to 113

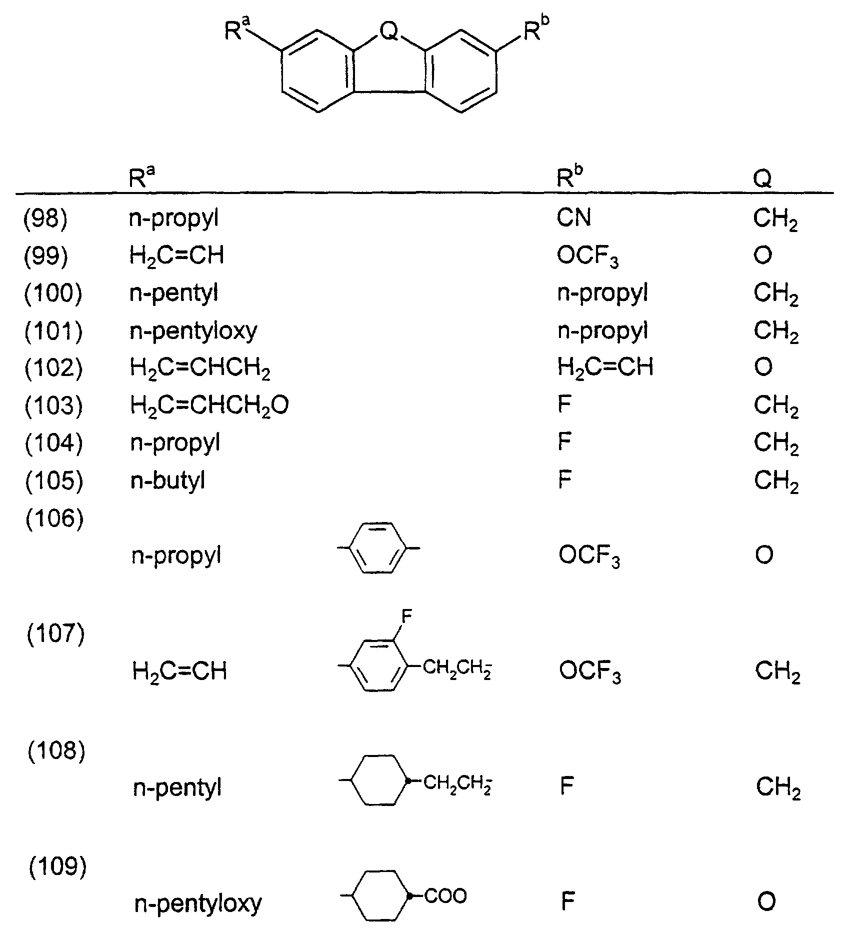

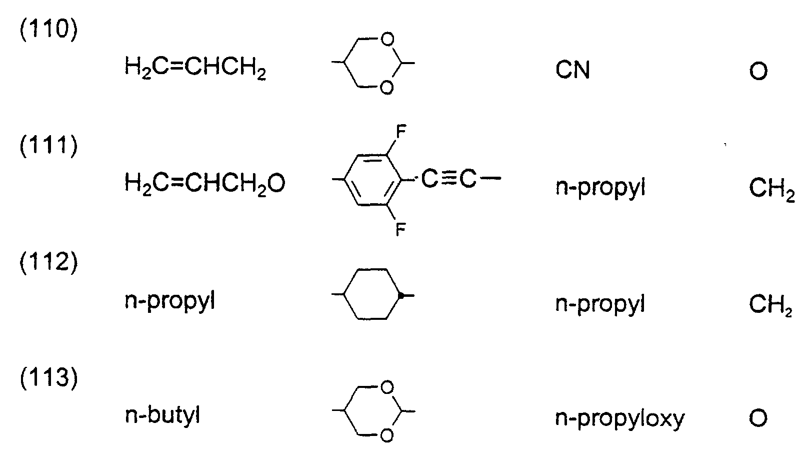

### Examples 114 to 124

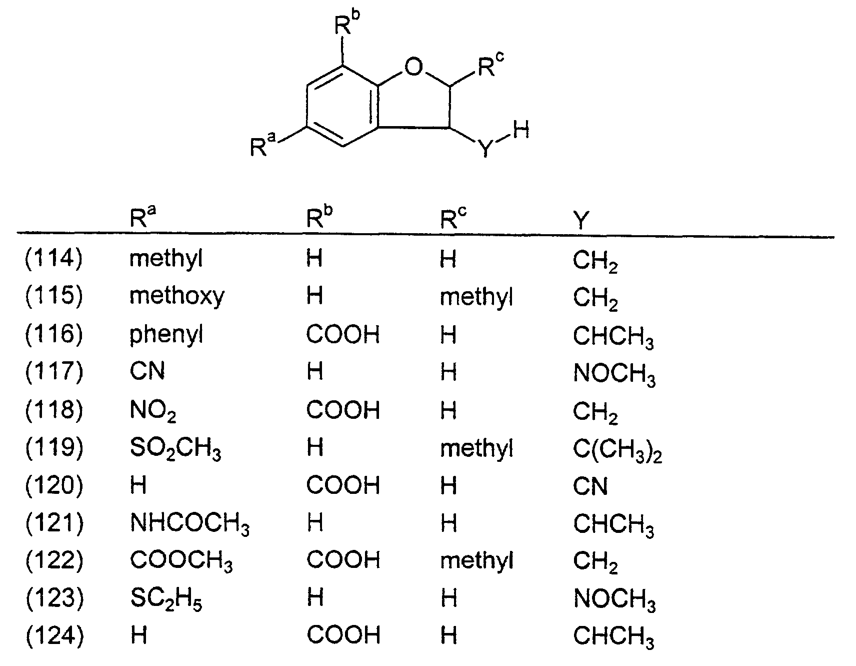

### Examples 125 to 134

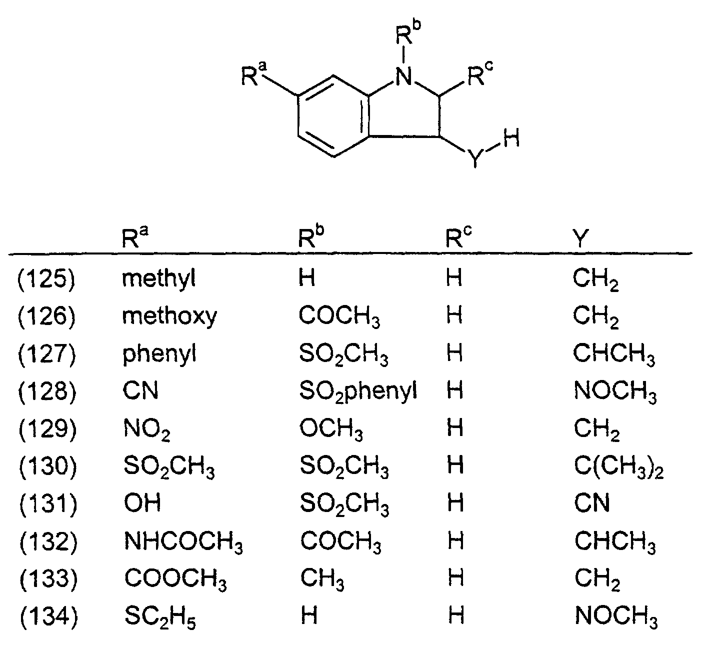

## Claims

1. A method for the conversion of alkenes or arenes with iodoalkenes, aryl iodides or arenediazonium salts in the presence of hypophosphorous acid or its derivatives, selected from the group consisting of salts of hypophosphorous acid, addition salts of hypophosphorous acid and nitrogen bases, dialkylphosphites, [1,3,2]dioxaphosphinane, [1,3,2]dioxaphospholane, dialkylphosphines, phospholane or phosphinane, and a radical initiator.

2. A method according to claim 1, **characterized in that** the reacting alkenes and/or arenes are part of the same compound.

3. A process according to claim 2 for the manufacture of compounds of formulae I and II 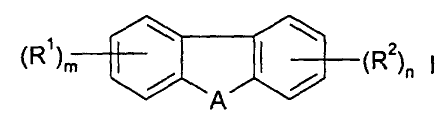 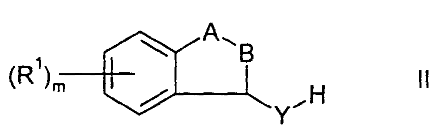 wherein
R¹, R² are each, independently of one another, H, F, Br, Cl, CN, NO₂, N(R³)₂, OR³, SR³, COOR³, NHCOR³, SF₅, SO₂R³ or Alkyl having 1 to 12 carbon atoms which is unsubstituted or at least monosubstituted by F, Br, Cl, CF₃ or CN, wherein it is also possible for one or more non-adjacent -CH₂-groups to be replaced, independently of one another, by -CH=CH-, -O-CH=CH-, -CH=CH-O-, -O-, -S-, -CO-, -O-CO- or -CO-O- or a mesogenic group of formula V
R⁰-A¹-Z¹-(A²-Z²-)ₚ- V
wherein
R⁰ H, F, Br, Cl, CN, NO₂, SF₅ or Alkyl having 1 to 12 carbon atoms which is unsubstituted or at least monosubstituted by F, Br, Cl, CF₃ or CN, wherein it is also possible for one or more non adjacent -CH₂-groups to be replaced, independently of one another, by -CH=CH-, -O-CH=CH-, -CH=CH-O-, -O-, -S-, -CO-, -O-CO- or -CO-O-,
A¹, A² are each, independently of one another,
a) trans-1,4-cyclohexylene in which one or two non-adjacent CH-groups may also be replaced by O or S,
b) 1,4-phenylene in which one or two CH-groups may also be replaced by N,
c) 1,3-cyclobutylene, 1,3-bicyclo(1,1,1)pentylene, 1,4-cyclohexenylene, 1,4-bicyclo(2,2,2)octylene, piperidine-1,4-diyl, naphthalene-2,6-diyl, decahydronaphthalene-2,6-diyl or 1,2,3,4-tetrahydronaphthalene-2,6-diyl,
wherein groups under a) and b) may be substituted by CN, F, Cl or Br,
Z¹, Z² are each, independently of one another, -CH₂CH₂-, -C≡C-, -CH₂O-, -OCH₂-, -CO-O-, -O-CO-, -CH=N-, -N=CH-, -CH₂S- or -SCH₂-, and
p is 0, 1, 2 or 3,
R³ H, alkyl or alkoxy having 1 to 12 carbon atoms or phenyl in which one or two CH-groups may also be replaced by N, which is unsubstituted or at least monosubstituted by F, Br, Cl, CF₃, CN, NO₂ or alkyl or alkoxy having 1 to 12 carbon atoms,
A, B are independently of one another, -O-, -S-, -N(R³)-, -N=C(R⁴)-, -C(R⁴)=N-, -C(R⁴)(R⁵)-, -C(R⁴)=C(R⁵)-, -C(R⁴)(R⁵)C(R⁶)(R⁷)-, -C(R⁴)(R⁵)-O-, -O-C(R⁴)(R⁵)-, -C(R⁴)(R⁵)-N(R³)- or -N(R³)-C(R⁴)(R⁵)- in such a way that heteroatoms are not linked directly to one another,
R⁴, R⁵, R⁶, R⁷ have independently of one another the meaning given for R¹ and R²,
Y CR⁴R⁵ or NR³
m, n 1, 2, 3 or 4,
by intramolecular reaction of the starting compounds III and IV 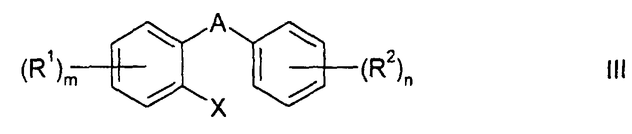 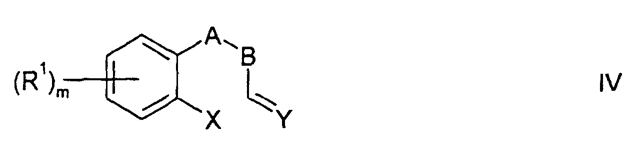 wherein
X is iodo or diazonium salt, and
R¹, R², A, B, Y, m and n have the meaning given above, in the presence of hypophosphorous acid or its derivatives and a radical initiator.

4. Process according to claim 1 to 3, **characterized in that** the starting compounds and hypophosphorous acid or its derivatives are heated in the presence of a radical initiator at a temperature of 30 to 200°C.

5. Process according to claim 1 to 4, wherein hypophosphorous acid its salts, dialkylphosphites or dialkylphosphines are used as reagent.

6. Process according to claim 1 to 5, wherein water or its mixture with organic solvents is used.

7. Process according to claim 1 to 6, wherein AIBN, dibenzoylperoxide, benzoylperoxide or di-tert-butylperoxide are used as radical initiators.

## Patentansprüche

1. Verfahren zur Umsetzung von Alkenen oder Arenen mit lodalkenen, Aryliodiden oder Arendiazoniumsalzen in Gegenwart von unterphosphoriger Säure oder deren Derivaten aus der Gruppe Salze der unterphosphorigen Säure, Additionssalze von unterphosporiger Säure und Stickstoffbasen, Dialkylphosphite, [1,3,2]-Dioxaphosphinan, [1,3,2]-Dioxaphospholan, Dialkylphosphine, Phospholan oder Phosphinan, sowie einem Radikalstarter.

2. Verfahren nach Anspruch 1, **dadurch gekennzeichnet, daß** die in die Umsetzung eingehenden Alkene und/oder Arene Teil derselben Verbindung sind.

3. Verfahren nach Anspruch 2 zur Herstellung von Verbindungen der Formeln I und II 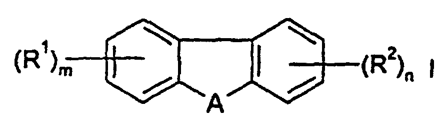 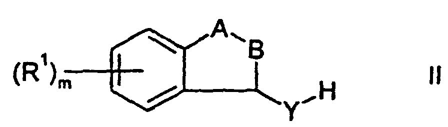 worin
R¹, R² jeweils unabhängig voneinander H, F, Br, Cl, CN, NO₂, N(R³)₂, OR³, SR³, COOR³, NHCOR³, SF₅, SO₂R³ oder Alkyl mit 1 bis 12 Kohlenstoffatomen, das unsubstituiert oder mindestens einfach durch F, Br, Cl, CF₃ oder CN substituiert ist, bedeuten, wobei auch eine oder mehrere nicht benachbarte -CH₂-Gruppen unabhängig voneinander durch -CH=CH-, -O-CH=CH-, -CH=CH-O-, -O-, -S-, -CO-, -O-CO- oder -CO-O- oder eine mesogene Gruppe der Formel V
R⁰-A¹-Z¹-(A²-Z²-)ₚ- V
ersetzt sein können, worin
R⁰ H, F, Br, Cl, CN, NO₂, SF₅ oder Alkyl mit 1 bis 12 Kohlenstoffatomen, das unsubstituiert oder mindestens einfach durch F, Br, Cl, CF₃ oder CN substituiert ist, bedeutet, wobei auch eine oder mehrere nicht benachbarte -CH₂-Gruppen unabhängig voneinander durch -CH=CH-, -O-CH=CH-, -CH=CH-O-, -O-, -S-, -CO-, -O-CO- oder -CO-O- ersetzt sein können,
A¹, A² jeweils unabhängig voneinander
a) trans-1,4-Cyclohexylen, wobei eine oder zwei nicht benachbarte CH-Gruppen auch durch O oder S ersetzt sein können,
b) 1,4-Phenylen, wobei ein oder zwei CH-Gruppen auch durch N ersetzt sein können,
c) 1,3-Cyclobutylen, 1,3-Bicyclo(1,1,1 )pentylen, 1,4-Cyclohexenylen, 1,4-Bicydo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl,
bedeuten, wobei die Gruppen von a) und b) durch CN, F, Cl oder Br substituiert sein können,
Z¹, Z² unabhängig voneinander -CH₂CH₂-, -C≡C-, CH₂O-, -OCH₂-, -CO-O-, -O-CO-, -CH=N-, -N=CH-, -CH₂S- oder -SCH₂- bedeuten, und
p 0, 1, 2 oder 3 bedeutet,
R³ H, Alkyl oder Alkoxy mit 1 bis 12 Kohlenstoffatomen oder Phenyl bedeutet, wobei eine oder zwei CH-Gruppen auch durch N, das unsubstituiert oder mindestens einfach durch F, Br, Cl, CF₃, CN, NO₂ oder Alkyl oder Alkoxy mit 1 bis 12 Kohlenstoffatomen substituiert ist, ersetzt sein können,
A, B unabhängig voneinander -O-, -S-, -N(R³), -N=C(R⁴)-, -C(R⁴)=N-, -C(R⁴)(R⁵)-, -C(R⁴)=C(R⁵)-, -C(R⁴)(R⁵)C(R⁶)(R⁷)-, -C(R⁴)(R⁵)-O-, -O-C(R⁴)(R⁵)-, -C(R⁴)(R⁵)-N(R³)- oder N(R³)-C(R⁴)(R⁵)- bedeuten, und zwar so, daß Heteroatome nicht direkt miteinander verbunden sind,
R⁴, R⁵, R⁶, R⁷ unabhängig voneinander die für R¹ und R² angegebene Bedeutung haben,
Y CR⁴R⁵ oder NR³ bedeutet und
m, n 1, 2, 3 oder 4 bedeuten,
durch intramolekulares Umsetzen der Ausgangsverbindungen III und IV 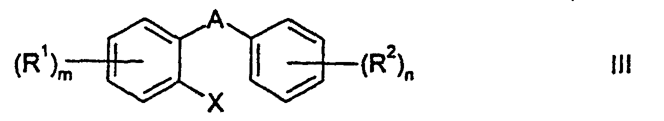 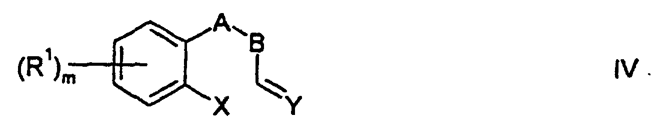 worin
X lod oder ein Diazoniumsalz bedeutet und
R¹, R², A, B, Y, m und n die oben angegebenen Bedeutungen aufweisen,
in Gegenwart von unterphosphoriger Säure oder ihren Derivaten sowie einem Radikalstarter.

4. Verfahren nach Anspruch 1 bis 3, **dadurch gekennzeichnet, daß** die Ausgangsverbindungen und die unterphosphorige Säure oder ihre Derivate in Gegenwart eines Radikalstarters auf eine Temperatur von 30 bis 200°C erhitzt werden.

5. Verfahren nach Anspruch 1 bis 4, wobei als Reagenz unterphosphorige Säure, ihre Salze, Dialkylphosphite oder Dialkylphosphine verwendet werden.

6. Verfahren nach Anspruch 1 bis 5, wobei Wasser oder eine Mischung von Wasser mit organischen Lösungsmitteln verwendet wird.

7. Verfahren nach Anspruch 1 bis 6, wobei als Radikalstarter AIBN, Dibenzoylperoxid, Benzoylperoxid oder Di-tert.-butylperoxid verwendet wird.

## Revendications

1. Procédé pour la conversion d'alcènes ou d'arènes avec des iodoalcènes, des iodures d'aryle ou des sels d'arènediazonium en présence d'acide hypophosphoreux ou de ses dérivés, choisis dans le groupe constitué par les sels d'acide hypophosphoreux, les sels d'addition d'acide hypophosphoreux et de bases azotées, les phosphites de dialkyle, le [1,3,2]-dioxaphosphinane, le [1,3,2]-dioxaphospholane, les dialkylphosphines, le phospholane et le phosphinane, et un amorceur de radicaux.

2. Procédé selon la revendication 1, **caractérisé en ce que** les alcènes et/ou arènes réagissant font partie du même composé.

3. Procédé selon la revendication 2, pour la préparation de composés de formules I et II 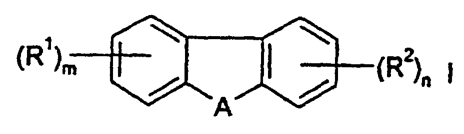 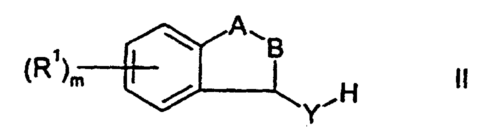 dans lesquelles
R¹, R² représentent chacun, indépendamment l'un de l'autre, H, F, Br, Cl, CN, NO₂, N(R³)₂, OR³, SR³, COOR³, NHCOR³, SF₅, SO₂R³ ou un groupe alkyle ayant de 1 à 12 atomes de carbone, qui est non substitué ou au moins monosubstitué par F, Br, Cl, CF₃ ou CN, dans lequel il est également possible qu'un ou plusieurs groupes -CH₂- non adjacents soient remplacés, indépendamment les uns des autres, par -CH=CH-, -O-CH=CH-, -CH=CH-O-, -O-, -S-, -CO-, -O-CO- ou -CO-O- ou un groupe mésogène de formule V
R⁰-A¹-Z¹-(A²-Z²-)ₚ- V
dans lequel
R⁰ représente H, F, Br, Cl, CN, NO₂, SF₅, ou un groupe alkyle ayant de 1 à 12 atomes de carbone, qui est non substitué ou au moins monosubstitué par F, Br, Cl, CF₃ ou CN, dans lequel il est également possible qu'un ou plusieurs groupes -CH₂- non adjacents soient remplacés, indépendamment les uns des autres, par -CH=CH-, -O-CH=CH-, -CH=CH-O-, -O-, -S-, -CO-, -O-CO- ou -CO-O-,
A¹, A² sont chacun, indépendamment l'un de l'autre,
a) un groupe *trans*-1,4-cyclohexylène dans lequel un ou deux groupes CH non adjacents peuvent également être remplacés par O ou S,
b) un groupe 1,4-phénylène dans lequel un ou deux groupes CH peuvent également être remplacés par N,
c) un groupe 1,3-cyclobutylène, 1,3-bicyclo(1.1.1)pentylène, 1,4-cyclohexénylène, 1,4-bicyclo(2.2.2)octylène, pipéridin-1,4-diyle, naphtalène-2,6-diyle, décahydronaphtalène-2,6-diyle ou 1,2,3,4-tétrahydronaphtalène-2,6-diyle,
les groupes en a) et b) pouvant être substitués par CN, F, Cl ou Br,
Z¹, Z² représentent chacun, indépendamment l'un de l'autre, -CH₂CH₂-, -C≡C-, -CH₂O-, -OCH₂-, -CO-O-, -O-CO-, -CH=N-, -N=CH-, -CH₂S- ou -SCH₂-, et
p est 0, 1, 2 ou 3.
R³ représente H, un groupe alkyle ou alcoxy ayant de 1 à 12 atomes de carbone, ou phényle dans lequel un ou deux groupes CH peuvent également être remplacés par N, qui est non substitué ou au moins monosubstitué par F, Br, Cl, CF₃, CN, NO₂ ou un groupe alkyle ou alcoxy ayant de 1 à 12 atomes de carbone,
A, B représentent,, indépendamment l'un de l'autre, -O-, -S-, -N(R³)-, -N=C(R⁴)-, -C(R⁴)=N-, -C(R⁴)(R⁵)-, -C(R⁴)=C(R⁵)-, -C(R⁴)(R⁵)C(R⁶)(R⁷)-, -C(R⁴)(R⁵)-O-, -O-C(R⁴)(R⁵)-, -C(R⁴)(R⁵)-N(R³)- ou -N(R³)-C(R⁴)(R⁵)- de telle façon que les hétéroatomes ne soient pas liés directement les uns aux autres,
R⁴, R⁵, R⁶, R⁷ ont indépendamment l'un de l'autre la signification donnée pour R¹ et R²,
Y représente CR⁴R⁵ ou NR³,
m, n représentent 1, 2, 3 ou 4,
par réaction intramoléculaire des composés de départ III et IV 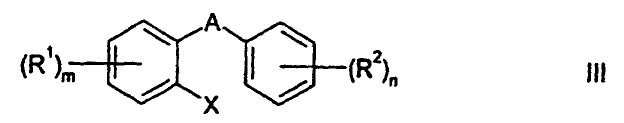 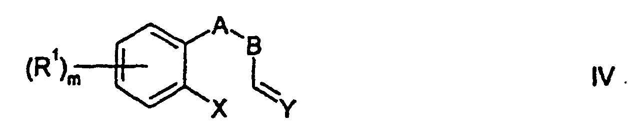 dans lesquels
X est un atome d'iode ou un sel de diazonium, et
R¹, R², A, B, Y, m et n ont les significations données plus haut,
en présence d'acide hypophosphoreux ou de ses dérivés et d'un amorceur de radicaux.

4. Procédé selon l'une quelconque des revendications 1 à 3, **caractérisé en ce que** les composés de départ et l'acide hypophosphoreux ou ses dérivés sont chauffés à une température de 30 à 200°C en présence d'un amorceur de radicaux.

5. Procédé selon l'une quelconque des revendications 1 à 4, dans lequel on utilise comme réactif l'acide hypophosphoreux, ses sels, des phosphites de dialkyle ou des dialkylphosphines.

6. Procédé selon l'une quelconque des revendications 1 à 5, dans lequel on utilise de l'eau ou un mélange d'eau et de solvants organiques.

7. Procédé selon l'une quelconque des revendications 1 à 6, dans lequel on utilise comme amorceurs de radicaux l'AIBN (azobisisobutyronitrile), le peroxyde de dibenzoyle, le peroxyde de benzoyle ou le peroxyde de di-tert-butyle.
